# EUROPEAN PATENT APPLICATION

(11) **EP 2 735 869 A1**
(43) Date of publication of application: **28.05.2014**
(21) Application number: 13180663.0
(22) Date of filing: 16.08.2013
(51) Int. Cl.: G01N 27/30

(54) **Bio-sensor**

(30) Priority: 23.11.2012 KR 20120133525
(71) Applicant: Samsung Electronics Co., Ltd, Gyeonggi-do 443-742 (KR)
(72) Inventor: Cho, Chul-Ho, 443-742 Gyeonggi-do (KR); Cho, Jae-Geol, 443-742 Gyeonggi-do (KR)
(74) Representative: Gover, Richard Paul

(57) **Abstract**

A bio-sensor configured with a lower substrate including a plurality of electrode portions, an insulating layer, a spacer layer, and an upper substrate provided on the spacer layer is provided. The bio-sensor includes a first sample injection portion provided between the upper substrate and the lower substrate along a longitudinal direction in which a sample is introduced and a second sample injection portion that is connected with the first sample injection portion in a direction substantially longitudinal direction, and formed larger than a sample inlet of the first sample injection portion.

## Description

### TECHNICAL FIELD

The present disclosure relates to a bio-sensor. More particularly, the present disclosure relates to a bio-sensor including an improved sample injection port.

### BACKGROUND

Generally, in a test that requires a liquid sample for clinical and environmental monitoring, most sensors used for measurement and analysis preferably have small sample capacity. A small sample capacity is required to allow accurate arrival in a measuring device in which a reaction occurs.

For example, there is increasing need to periodically measure the amount of glucose in the blood (blood sugar) in the diagnosis and prevention of diabetes. Such blood sugar may be easily measured using a blood sugar measuring device that collects a sample from a patient using a bio-sensor in the form of a blood sugar strip. The blood sugar measuring device measures a blood sugar value using an electric signal generated through an electro-chemical reaction between the collected sample and a chemical material in the bio-sensor.

The bio-sensor is an electrode system including a plurality of electrodes on an insulating substrate formed by a method such as printing an enzyme reaction layer composed of a hydrophilic high-polymer, oxidoreductase, and an electronic acceptor on the formed electrode system.

Once a sample that includes glucose is injected into the enzyme reaction layer through a sample injection port of the bio-sensor, the enzyme reaction layer dissolves the sample and an enzyme of the sample reacts with the enzyme reaction layer to oxidize the glucose, such that an electronic acceptor is reduced.

By measuring an oxidation current acquired by electro-chemical oxidization of the reduced electronic acceptor, the concentration of the glucose in the sample may be obtained.

In the bio-sensor, a sufficient sample is required for the reaction and must be filled in an accurately fixed area. To this end, the bio-sensor usually uses a structure in which a narrow flow path is formed to induce a capillary action.

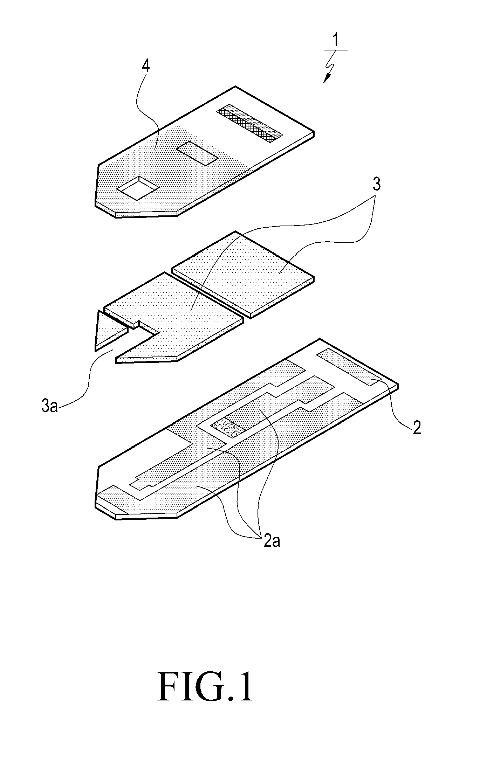

Such a bio-sensor 1 includes a lower substrate 2 having electrodes 2a, an intermediate substrate 3 including a sample injection portion 3a, and an upper substrate 4, as illustrated in FIG. 1.

FIG. 1 is an exploded perspective view of a bio-sensor according to the related art.

A three-dimensional (3D) shape of the bio-sensor 1 is mostly a rectangular parallelepiped in the shape of "|" or "-". A user injects a sample into the sample injection portion provided at an end portion to guide the flow of the sample into the bio-sensor.

As such, an example of the sample injection portion of the bio-sensor may be classified into a "|"-shape type and a "-"-shape type.

The foregoing two types of sample injection portions are structured such that a tip portion of a straight-line sample injection port is perforated to form a capillary tube. A required blood volume of the blood sugar measuring strip is less than 1 µl, and if this volume of blood is collected from a fingertip, the blood takes the form of a droplet having a diameter of about 2 mm. The capillary tube shape is a rectangular parallelepiped with a width and a length in millimeter range, but a height of 100 µm. Thus, it is not easy for a patient to accurately inject a droplet having a diameter of about 2 mm into a fixed cross-sectional area having a width of 3 mm or less and a height of 100 µm. If the sample is not correctly injected, the user's blood may be collected several times. Moreover, the blood sugar strip may be contaminated and, thus, has to be discarded.

That is, as the size of the capillary tube of the sample injection port increases, more blood is required to fill the capillary tube, which may not be pleasant to the user. As a result, a conventional blood sugar strip has been designed and manufactured to minimize required blood volume by reducing the size of the capillary tube. Although the required sample volume is reduced because the size of the capillary tube is reduced, the injection position and the shape of the sample injection portion are also reduced in size, thereby making it difficult for the user to inject the sample into the size-reduced sample injection portion.

Therefore, to overcome the foregoing problem, there is a need for a separate sample injection portion that is larger than a conventional sample injection portion to improve the injection of a small-volume sample.

The above information is presented as background information only to assist with an understanding of the present disclosure. No determination has been made, and no assertion is made, as to whether any of the above might be applicable as prior art with regard to the present disclosure.

### SUMMARY

Aspects of the present disclosure are to address at least the above-mentioned problems and/or disadvantages and to provide at least the advantages described above.

Accordingly, various aspects of the present disclosure provide a bio-sensor in which a second sample injection portion is formed larger than a sample inlet of a first sample injection portion, facilitating sample injection into a device through a larger sample injection portion and increasing successful sample injection, thus preventing the inconvenience of multiple sample collection attempts and also preventing contamination of the device.

Other objects to be provided in the present disclosure may be understood by various embodiments described below.

According to an aspect of the present disclosure, a bio-sensor configured with a lower substrate including a plurality of electrode portions, an insulating layer, a spacer layer, and an upper substrate provided on the spacer layer is provided. The bio-sensor includes a first sample injection portion provided between the upper substrate and the lower substrate along a longitudinal direction in which a sample is introduced and a second sample injection portion that is connected with the first sample injection portion in a direction substantially perpendicular to the longitudinal direction, and formed larger than a sample inlet of the first sample injection portion.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features and advantages of certain various embodiments of the present disclosure will be more apparent from the following description taken in conjunction with the accompanying drawings, in which:

FIG. 1 is an exploded perspective view of a bio-sensor according to the related art;

FIG. 2 is an exploded perspective view of a bio-sensor according to an embodiment of the present disclosure;

FIG. 3 is a perspective view illustrating an assembly state of a bio-sensor according to an embodiment of the present disclosure;

FIG. 4 is a top view illustrating a state before a bio-sensor operates according to an embodiment of the present disclosure;

FIG. 5 is a top view illustrating an operating process of a bio-sensor according to an embodiment of the present disclosure; and

FIG. 6 is a top view illustrating a state after a bio-sensor operates according to an embodiment of the present disclosure.

Throughout the drawings, like reference numerals will be understood to refer to like parts, components, and structures.

### DETAILED DESCRIPTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the present disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

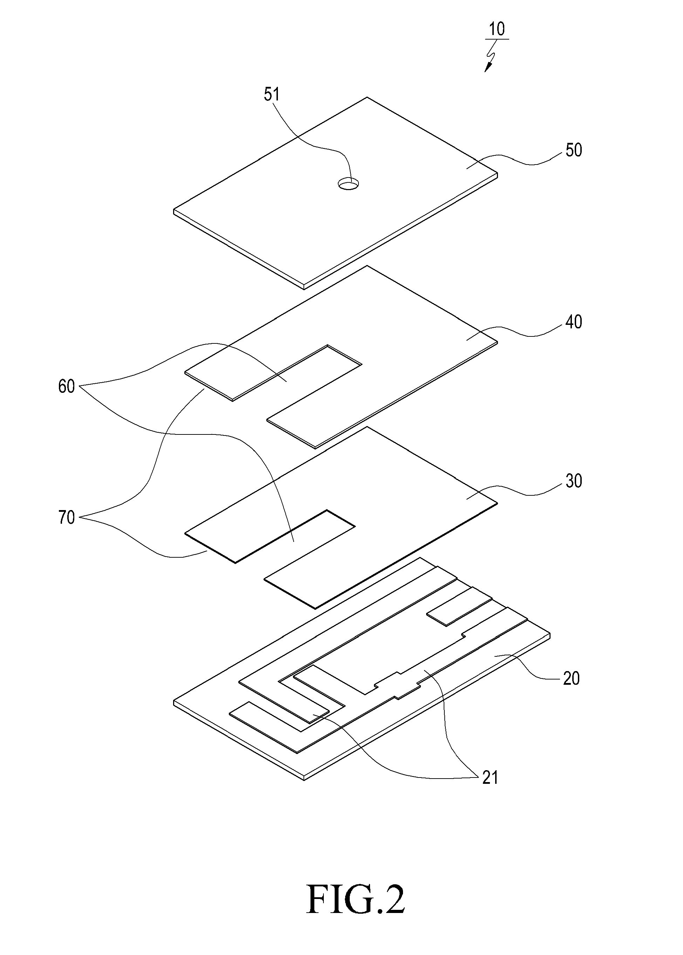

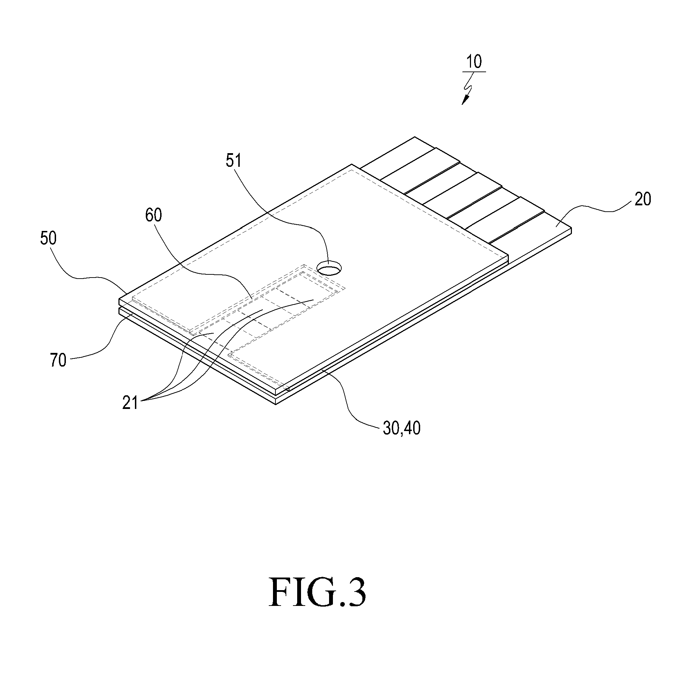

FIGS. 2 and 3 illustrate a structure of a bio-sensor according to an embodiment of the present disclosure.

Referring to FIGS. 2 and 3, the bio-sensor 10 may include a lower substrate 20 including a plurality of electrode portions 21, an insulating layer 30, a spacer layer 40, and an upper substrate 50 provided on the spacer layer 40. The lower substrate 20 is provided to include the electrode portions 21 including a reference electrode, an auxiliary electrode, and an operating electrode (not shown). The insulating layer 30 is provided on the lower substrate 20 to electrically insulate the electrode portions 21. The spacer layer 40 is provided on the insulating layer 30 to include an enzyme portion (not shown) including materials that can electro-chemically react with a sample A1 (not shown). The upper substrate 50 that is disposed over the spacer layer 40 includes an air discharge hole 51 to allow movement of the introduced sample A1. A first sample injection portion 60 is provided in a longitudinal direction between the lower substrate 20 and the upper substrate 50 to allow introduction of the sample A1. A second sample injection portion 70 is placed between the lower substrate 20 and the upper substrate 50 that is connected with the first sample injection portion 60 and is generally configured to be substantially perpendicular to with respect to the longitudinal direction. The second sample injection portion 70 is formed larger than a sample inlet of the first sample injection portion 60.

In this way, the second sample injection portion 70 is formed larger than the sample inlet of the first sample injection portion 60 such that, if a contact portion for user's injection of the sample A1 is large, the second sample injection portion 70 facilitates collection of the sample A1.

The first sample injection portion 60 and the second sample injection portion 70 are preferably formed by removing a portion of the insulating layer 30 and the spacer layer 40. That is, the upper substrate 50, the lower substrate 20, the insulating layer 30, and the spacer layer 40 are sequentially coupled in a stacked manner and, at the same time, the first sample injection portion 60 and the second sample injection portion 70 are formed.

Moreover, the first sample injection portion 60 and the second sample injection portion 70 are formed in the shape of "T". Herein, the first sample injection portion 60 and the second sample injection portion 70 may also be configured in other suitable shapes (for example, an arrow (←) shape, an "L" shape, and a "Y" shape).

The second sample injection portion 70 is preferably formed in a hemispheric shape. However, the second sample injection portion 70 may also be in other suitable shapes (for example, an oval shape).

An angle between the first sample injection portion 60 and the second sample injection portion 70 is 45° - 135°, preferably 60° - 120°, and most preferably 75° - 105°.

The volume of the sample A1 is 0.1 µl - 2.0 µl, preferably 0.1 µl - 1.0 µl, and most preferably 0.3 µl - 0.7 µl.

Generally, if the volume of the sample A1 is less than 0.1 µ*l*, the volume of the sample A1 is too small and an error range of the bio-sensor 10 exists such that accurate measurement may not be guaranteed. On the other hand, if the volume of the sample A1 exceeds 3.0 µ*l*, a problem may occur due to the excessive sample A1.

Thus, the volume of the sample A1 is most preferably 0.3 µ*l -* 0.7 µ*l*, as mentioned above.

A material for the first sample injection portion 60 and the second sample injection portion 70 is preferably a rigid material such as a ceramic material, a glass plate, and a high-polymer material. The material for the first sample injection portion 60 and the second sample injection portion 70 may also be a material other than the foregoing material.

Moreover, the high-polymer material may comprise polyester, a polyvinyl chloride, and a polycarbonate material.

The first sample injection portion 60 and the second sample injection portion 70 may be treated with oxygen plasma and coated with an interfacial active agent.

A material of the electrode portions 21 may include one or more of epoxy, palladium, copper, gold, white gold, iridium, silver, silver chloride, and carbon. However, the material of the electrode portions 21 may also be a material other than the foregoing material.

The electrode portions 21 may be attached to the lower substrate 20 using suitable methods such as, for example, one or more of screen printing, vapor deposition, etching, and conductive tapes.

The electrode portions 21 include a reference electrode (not shown), an auxiliary electrode (not shown), and an operating electrode (not shown).

The reference electrode and the auxiliary electrode measure a resistance of the sample A1 injected through the first sample injection portion 60 and the second sample injection portion 70.

The operating electrode detects the amount of current and measures a blood sugar level of the sample A1 by using the detected amount of current.

That is, a resistance between the reference electrode and the auxiliary electrode is measured after the injection of the sample A1, and a time is counted from the detection of the amount of current in the operating electrode to sensing of a change in the resistance between the reference electrode and the auxiliary electrode. Using the counted time, it is determined whether the injection of the sample A1 is normal. In this state, the measured blood sugar level is correctly measured according to the measured resistance between the reference electrode and the auxiliary electrode.

Herein, assembly of the bio-sensor 10 will be described with reference to FIGS. 2 and 3. The insulating layer 30 is placed on the lower substrate 20 including the multiple electrode portions 21, the spacer layer 40 is stacked on the insulating layer 30, and the upper substrate 50 is stacked on the spacer layer 40.

The spacer layer 40 includes an enzyme portion (not shown) that includes materials that may electro-chemically react with the sample A1.

As such, the upper substrate 50, the lower substrate 20, the insulating layer 30, and the spacer layer 40 are coupled in a stacked manner and at the same time, the first sample injection portion 60 and the second sample injection portion 70 are formed between the upper substrate 50 and the lower substrate 20.

Portions of the insulating layer 30 and the spacer layer 40 are removed to form the first sample injection portion 60 and the second sample injection portion 70.

That is, a portion of the insulating layer 30 and the spacer layer 40 is removed in a longitudinal direction to form the first sample injection portion 60. Then, a portion of the insulating layer 30 and the spacer layer 40 is removed generally in a direction that is perpendicular with respect to the longitudinal direction to form the second sample injection portion 70.

Therefore, the first sample injection portion 60 is provided in the longitudinal direction, and the second sample injection portion 70 is connected with the first sample injection portion 60 that is generally perpendicular to the longitudinal direction. Further, the second sample injection portion 70 is formed larger than the sample inlet of the first sample injection portion 60.

The operation of the bio-sensor 10 in this state will be described in more detail.

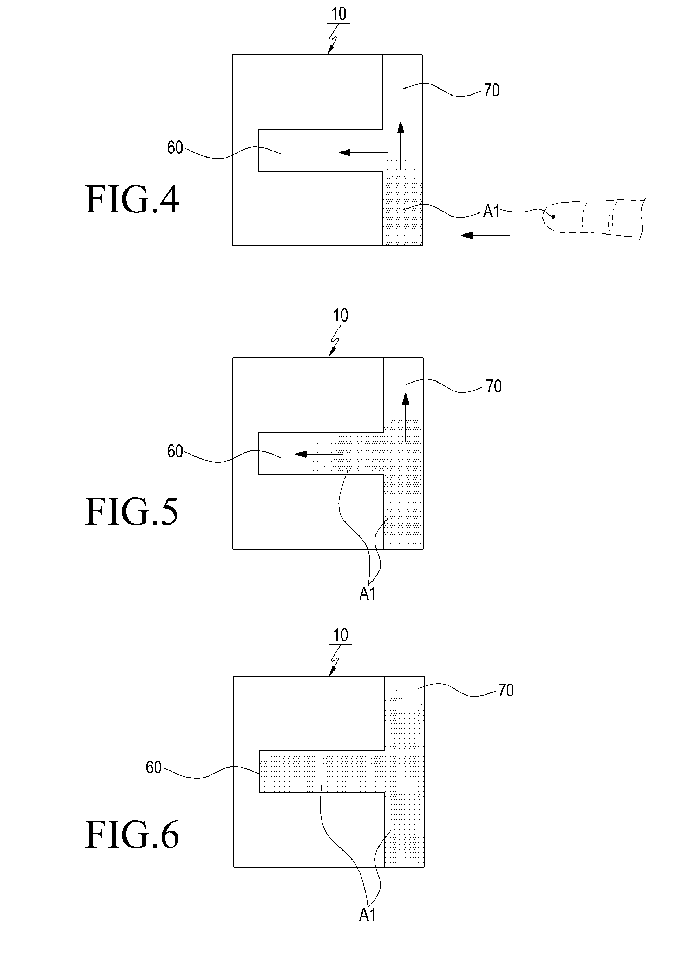

FIG. 4 is a top view illustrating a state before a bio-sensor operates according to the present disclosure.

Referring to FIG. 4, the sample A1 of a volume of 0.3 µ*l -* 0.7 µ*l* is injected into the second sample injection portion 70. The second sample injection portion 70 is formed larger than the sample inlet of the first sample injection portion 60. The larger inlet of the second sample injection portion 70 is configured such that the sample A1 may flow into the second sample injection portion 70.

FIG. 5 is a top view illustrating an operating process of a bio-sensor according to an embodiment of the present disclosure.

Referring to FIG. 5, the sample A1 that is injected into the second sample injection portion 70 moves to a center portion of the second sample injection portion 70 and continues moving to the first sample injection portion 60.

FIG. 6 is a top view illustrating a state after a bio-sensor operates according to an embodiment of the present disclosure.

Referring to FIG. 6, the sample A1 flows through the first sample injection portion 60 and moves to the enzyme portion (not shown) included in the spacer layer 40.

Referring to FIG. 3, the air discharge hole 51 formed on the upper substrate 50 discharges air to allow the sample A1 to flow into the first sample injection portion 60 and the second sample injection portion 70.

In other words, the injected sample A1 flows through the first sample injection portion 60, into the second sample injection portion 70, and into the enzyme portion, which electro-chemically reacts with the sample A1 through the materials included therein (not shown).

Once the enzyme portion of the bio-sensor in the insulating layer 30 electro-chemically reacts with the sample A1, the blood sugar measuring device (not shown) receives an electric signal from the operating electrode, the reference electrode, and the auxiliary electrode to measure the blood sugar level and displays the measured blood sugar level on a display unit (not shown).

More specifically, once power is supplied to the operating electrode, an amplifier (not shown) of the blood sugar measuring device detects the amount of current flowing through the operating electrode based on power supply and outputs the detected amount of current as an analog voltage.

An Analog-to-Digital (A/D) converter (not shown) of the blood sugar measuring device converts the voltage into a digital signal and transmits the digital signal to a controller (not shown).

A resistance measuring unit of the blood sugar measuring device measures a resistance between the auxiliary electrode and the reference electrode and transmits the measured resistance to the controller.

By controlling the overall operation of the blood sugar measuring device, the blood sugar measuring device displays the measured blood sugar level on the display unit.

That is, in the conventional bio-sensor, the volume of a sample is reduced as the size of the capillary tube of the sample inlet is reduced. Thus, the size of the position in which injection is to be performed and the size of the sample injection portion are also reduced, making it difficult for the user to inject the sample into the size-reduced injection portion.

To overcome such a disadvantage, the present disclosure provides the second sample injection portion 70 formed larger than the first sample injection portion 60 to facilitate injection of the sample (A1 of FIG. 5), such that the user may be easily inject the sample A1 through a larger sample injection portion than the conventional one, and thus collection of the sample A1 of the product may be improved.

Meanwhile, the bio-sensor according to an embodiment of the present disclosure may be implemented in a blood sugar measuring device. However, the present disclosure may also be implemented in electro-chemical testing sensors in various forms for collecting and analyzing a blood sample (for example, a portable testing device and so forth).

The above-described bio-sensor according to the present disclosure is not limited by the foregoing embodiment and drawings, and it would be obvious to those of ordinary skill in the art that various substitutions, changes, and modifications may be made within the technical scope of the present disclosure.

While the present disclosure has been shown and described with reference to various embodiments thereof, it will be understood by those skilled in the art that various changes in form and details may be made therein without departing from the spirit and scope of the present disclosure as defined by the appended claims and their equivalents.

## Claims

1. A bio-sensor(10) configured with a lower substrate(20) comprising a plurality of electrode portions(21), an insulating layer(30), a spacer layer(40), and an upper substrate(50) provided on the spacer layer, the bio-sensor comprising:
a first sample injection portion(60) provided between the upper substrate and the lower substrate along a longitudinal direction in which a sample(A1) is introduced; and
a second sample injection portion(70) that is connected with the first sample injection portion in a direction substantially perpendicular to the longitudinal direction, and formed larger than a sample inlet of the first sample injection portion(60).

2. The bio-sensor of claim 1, wherein the first sample injection portion(60) and the second sample injection portion(70) are formed by removing portions of the insulating layer(30) and the spacer layer(40).

3. The bio-sensor of claim 1, wherein the first sample injection portion(60) and the second sample injection portion(70) have the shape of "T".

4. The bio-sensor of claim 1, wherein the second sample injection portion(70) has a hemispheric shape.

5. The bio-sensor of claim 1, wherein an angle between the first sample injection portion(60) and the second sample injection portion(70) is 45° - 135°.

6. The bio-sensor of claim 1, wherein an angle between the first sample injection portion(60) and the second sample injection portion(70) is 75° - 105°.

7. The bio-sensor of claim 1, wherein a volume of the sample(A1) is 0.1 µ*l* - 2.0 µ*l*.

8. The bio-sensor of claim 1, wherein a volume of the sample(A1) is 0.3 µ*l* - 0.7 µ*l.*

9. The bio-sensor of claim 1, wherein a material for the first sample injection portion(60) and the second sample injection portion(70) is one or more of a ceramic, a glass, and a polymer material.

10. The bio-sensor of claim 9, wherein the high-polymer material comprises one or more of a polyester, a polyvinyl chloride, and a polycarbonate.

11. The bio-sensor of claim 1, wherein the first sample injection portion(60) and the second sample injection portion(70) are treated with oxygen plasma and coated with an interfacial active agent.

12. The bio-sensor of claim 1, wherein a material for the electrode portions(21) comprises one or more of epoxy, palladium, copper, gold, white gold, iridium, silver/silver chloride, and carbon.

13. The bio-sensor of claim 1, wherein the electrode portions(21) are attached to the lower substrate(20) via one or more of screen printing, vapor deposition, etching, and conductive tapes.

14. The bio-sensor of claim 1, wherein the electrode portions(21) comprise a reference electrode, an auxiliary electrode, and an operating electrode.
